Europäisches Patentamt
European Patent Office
Office européen des brevets

(11) Publication number: 0 215 669
A2

(12) # EUROPEAN PATENT APPLICATION

(21) Application number: 86307115.5

(22) Date of filing: 16.09.86

(51) Int. Cl.4: H 01 L 41/08
C 12 Q 1/04, G 01 N 33/53

(30) Priority: 17.09.85 JP 204949/85
10.03.86 JP 51657/86
10.03.86 JP 51658/86
21.05.86 JP 116626/86
06.06.86 JP 131481/86
06.06.86 JP 131482/86

(43) Date of publication of application:
25.03.87 Bulletin 87/13

(84) Designated Contracting States: DE FR GB SE

(71) Applicant: SEIKO INSTRUMENTS & ELECTRONICS LTD.
31-1, Kameido 6-chome
Koto-ku Tokyo 136 (JP)

(72) Inventor: Karube, Isao
11-8, Fujimi-cho, 4-chome
Tachikawa-shi Tokyo (JP)

Muramatsu, Hiroshi c/o Seiko Instruments
& Electronics Ltd. 31-1, Kameido 6-chome
Koto-ku Tokyo (JP)

(74) Representative: Caro, William Egerton et al
J. MILLER & CO. Lincoln House 296-302 High Holborn
London WC1V 7JH (GB)

(54) **Analytical device and method for analysis of biochemicals, microbes and cells.**

(57) An analytical device and a method for the analysis of biochemicals, microbes and cells using a piezoelectic crystal biosensor, utilises the resonant frequency change caused by weight change on the surface of the piezoelectric crystal on which receptor materials is immobilised.

Four piezoelectric crystals 161 are mounted in a common cell 164. Each crystal 161 has a pair of electrodes 162 connected by leads 163 to an oscillator circuit 166. The electrodes 162 of three of the crystals 161 are treated to deposit different lectins thereon and those of the fourth left untreated as a reference. A peristaltic pump 169 draws liquid through pipes 165 and cell 164 from a valve 1610 which is solenoid operated to draw from a blood sample supply 1612 or from another valve 1611 drawing from either a phosphate buffer solution reservoir 1613 or a removal agent reservoir 1614. The circuit 166 is connected to a frequency counter 167 whose outputs are fed to a computer 168 which controls the pump 169 and valves 1610 and 1611.

The crystals 161 are first resonated to establish their resonant frequencies. A blood sample is then drawn through the cell 164 by pump 169. Type A erythrocytes are attracted to one of the lectins, types A and B to another, and type O to the third. Phosphate buffer is then drawn through by adjustment of valves 1610 and 1611. Resonant frequencies are measured again and compared with those originally obtained. From the changes observed, determination of type is made by the computer. Removal agent is then drawn through to remove remaining erythrocytes and phosphate buffer drawn through to prepare for further samples.

FIG.16

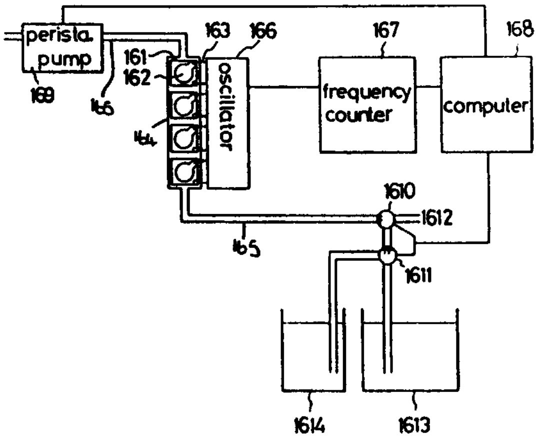


EP 0 215 669 A2

**Description**

ANALYTICAL DEVICE AND METHOD FOR ANALYSIS OF BIOCHEMICALS, MICROBES AND CELLS

The present invention relates to analytical devices and methods for the analysis of biochemicals, microbes and cells. Various methods for the analysis of biochemicals, microbes and cells have included liquid chromatography, electrophoresis, direct observation of agglutination with the naked eye, and applying amplification with enzymes, fluorescent chemicals and radio isotopes (EIA, FIA, RIA).

Particularly, for the detection of a pathogenic microorganism, a conventional method involves adding an antibody solution dropwise to a microbial suspension and observing the agglutination with the naked eye by using an agglutination plate. The problem with the agglutination method is that for agglutination to be judged by the naked eye requires one highly skilled in the art. In addition, this method gives a poor quantitative result and can hardly be automated.

Recently a system for the judgment of agglutination has been developed, employing an image processing system equipped with a microscope. The image processing system is of large size and considerable cost.

Another method involving applied electrochemical determination and immuno reaction, is known. In this method, a microorganism is bound to an antibody immobilised on an organic film and concentration is determined by measuring a change in the membrane potential. This method is troublesome in operation and can hardly be automated.

On the other hand, for immunoassay, a surface acoustic wave (SAW) device has been developed, and is reported in the following document: J.E. Roederer and G.J. Bastiaans, Anal. Chem. 1983, 55, 2333 - 2336. This reference discloses that determination of human IgG is possible applying specific adsorption between immobilised goat anti-human IgG and human IgG at the surface of the SAW device.

These conventional methods have some disadvantages in that they give poor sensitivity and are troublesome in operation.

Immunoassay methods such as EIA, FIA and RIA, although they have a good sensitivity, are also troublesome in operation.

The SAW device, for human IgG, only gives a rough determination of the range of human IgG:0.0225 - 2.25 mg/ml.

The present invention seeks to overcome these disadvantages and to provide analytical devices and methods which are simple to operate, sensitive and which lend themselves to automation.

It is one object of the present invention to provide a piezoelectric crystal biosensor, which can detect biochemicals, microbes and cells, in particular pathogenic microorganisms, and can selectively determine the concentration of the same.

Another object of the invention is to provide a highly sensitive concentration determination method for the piezoelectric crystal biosensor.

A further object of the invention is to provide an improved piezoelectric crystal biosensor, which can analyse the constituents of biochemicals.

A yet further object of the present invention is to provide a measuring system utilising a piezoelectric crystal biosensor which has high sensitivity and short operation time, such method being inexpensive, automatic in operation and capable of repetition.

According to one aspect of the invention, an analytical device for biochemicals, microbes and cells, comprises a piezoelectric crystal sensor and means for measuring the resonant frequency of the sensor, the sensor exhibiting a resonant frequency change caused by weight change on the surface of the piezoelectric crystal due to the biochemical, microbe or cell to be analysed.

Such an analytical device may comprise a plurality of piezoelectric crystal sensors and means for measuring the resonant frequencies of the sensors, at least one of the sensors exhibiting a resonant frequency change caused by weight change on the surface of the piezoelectric crystal due to the biochemical, microbe or cell to be analysed.

From another aspect, the invention includes a method for the analysis of biochemicals, microbes and cells, comprising (a) applying adsorbent biochemical or organic compound to a piezoelectric crystal biosensor surface; (b) allowing the biochemical or organic compound to adsorb biochemicals, microbes or cells to be analysed; (c) measuring the resonant frequency change caused by a weight change on the piezoelectric crystal surface; and (d) detecting the biochemicals, microbes or cells, and determining concentration of the biochemicals, microbes or cells from a calibration curve obtained previously.

From a further aspect, the invention is a method for the analysis of biochemicals, microbes and cells using a piezoelectric crystal biosensor, comprising immobilising at least one material which can adsorb biochemicals, microbes or cells on the surface of the piezoelectric crystal, allowing immobilised material to adsorb the biochemicals, microbes or cells to be analysed, and analysing the constituents of the biochemicals, microbes or cells on the basis of the ability of the immobilised material to adsorb the biochemicals, microbes or cells.

From a yet further aspect, a method according to the invention for the analysis of biochemicals using a piezoelectric crystal biosensor, comprises immobilising at least one material which can adsorb biochemicals on the surface of the piezoelectric crystal, allowing the immobilised material to adsorb the biochemical to be analysed, flowing an eluent over the surface and analysing the constituents of the biochemical from a change in the resonant frequency of the crystal before and after the elution.

Whilst some aspects of the present invention are defined by the appended claims, it will be appreciated that other, and more or less restricted inventions are disclosed herein and are derivable from the disclosure hereof.

The invention employs the phenomenon of resonant frequency change of a piezoelectric crystal induced by weight change on the surface of the crystal. The surface of the crystal is provided with material which will attract and hold biochemicals, microbes or cells to be detected. If the material is such as to attract only specific biochemicals, microbes or cells, the existence of weight change is an indication of the presence of the specific biochemical, microbe or cell, and the amount of weight change is an indication of its concentration. If the material is such as to attract a plurality of specific biochemicals, microbes or cells, differential removal of individual specific biochemicals, microbes or cells, will indicate by the resultant weight change the presence and concentration of the individual specific biochemicals, microbes or cells.

The present invention has made it possible to detect biochemicals, microbes and cells and to determine the concentration of the same by continuous operation of immobilising or modifying a biochemical or organic compound on the surface of the electrode of a piezoelectric crystal, specifically binding reaction of the biochemical or organic compound with biochemicals to cause weight change on the electrode surface of the piezoelectric crystal, and measuring the resonant frequency change, induced by weight change.

The invention is described below with reference to the accompanying drawings, in which:-

Figure 1 is a perspective view of a piezoelectric crystal biosensor forming a device according to the invention,

Figure 2 is a sectional view of the biosensor along the line II-II of Figure 1;

Figure 3 is a block diagram of a frequency measurement system for the biosensor of Figure 1;

Figure 4 is a graph showing the correlation of Candida (C. albicans) concentration and frequency change with the biosensor of Figure 1;

Figure 5 is a graph showing the correlation of Saccaromyces (S. cerevisiae) concentration and frequency change with the piezoelectric crystal biosensor;

Figure 6 is a perspective view of a biosensor similar to that of Figure 1 within a liquid flow cell;

Figure 7 is a block diagram of a biosensor system incorporating the device of Figure 6,

Figure 8 is a graph showing the correlation of human IgG concentration and frequency change;

Figure 9 and Figure 10 show, to an enlarged scale, the surface of a biosensor crystal to illustrate the operation thereof;

Figure 11 shows diagrammatically the surface of a biosensor crystal during different stage of operation;

Figure 12(a) is a graph showing the resonant frequency before and after reaction with mouse gamma-globulin and at different later stages of elution;

Figure 12(b) is a graph showing the relation between each pH of buffer solution and the difference of the resonant frequency;

Figure 13 is a graph showing the relation between each pH of buffer solution and the difference of the resonant frequency during analysis of the constituents of human IgG;

Figure 14 is a perspective view of a device for analysis of biochemicals according to the invention with a plurality of AT-cut piezoelectric crystals within a common cell;

Figure 15 is a perspective view of a similar device with GT-cut piezoelectric crystals; and

Figure 16 is a block diagram of a multiple piezoelectric biosensor analytical system used as an ABO blood type sensor.

I. Piezoelectric crystal biosensor for the detection and concentration determination of a pathogenic microbe.

The present invention enables the detection of a microorganism, in particular a pathogenic microbe, and to determine the concentration of the same. This is achieved by immobilising an antibody on the surface of a piezoelectric crystal biosensor to act as a receptor for the microorganism to be detected, binding the microorganism in various concentrations to the antibody thereby tocause weight changes on the surface of electrodes of the piezoelectric crystal biosensor, and measuring the changes in the resonant frequency of the piezoelectric crystal biosensor induced by the weight changes.

The amount of microorganisms, bound to an immobilised antigen through an antigen-antibody reaction in a definite time, depends on the concentration of the microorganism. Thus the resonant frequency change of the piezoelectric crystal biosensor depends on the concentration of the microorganism and a calibration curve is obtained of resonant frequency change against concentration.

The piezoelectric crystal of a biosensor thus calibrated and having a known resonant frequency, is immersed in microbial suspension thereby to induce an antigen-antibody reaction for a definite time. The resonant frequency is then measured again. From this, the concentration of the microorganism can be determined from the resonant frequency change based on a calibration curve with is previously formed.

EXAMPLE 1

A piezoelectric crystal biosensor 8 (Figures 1 and 2) is used for detection and concentration determination of Candida (Candida albicans (hereinafter referred to as C. albicans)) which is a pathogenic yeast. The biosensor 8 according to the invention includes an AT-cut piezoelectric crystal 1 of 9 MHz which has electrodes 2 on both sides on which electrodes C. albicans antibody 3 was immobilised. The electrodes 2 are connected to lead wires 4 by which they are connectible to an oscillator circuit 6 (Figure 3). The oscillator circuit 6 is connected to a frequency counter or meter 7. During application of a sample C. albicans solution to the biosensor 8, C. albicans 5 (Figure 2) is captured by the C. albicans antibody 3, both shown greatly enlarged and diagrammatically.

The C. albicans antibody 3 was immobilised on the surface of the electrodes 2 in the following manner. The surface of each electrode 2 of the piezoelectric crystal 1 was plated with palladium and the electrode 2 was electrolytically oxidised. After allowing the piezoelectric crystal to react in a 2% solution of gamma-aminopropyl triethoxy silane in acetone for an hour, washing the crystal with water and drying, the lead wires 4 were connected to the oscillator circuit 6 and the resonant frequency $F_1$ was measured by the frequency counter 7. Subsequently, the crystal was allowed to react in a 5% aqueous solution of glytaraldehyde for three hours and C. albicans antibody was added dropwise on the electrodes and allowed to stand for 30 minutes to thereby immobilise the antibody thereon. Then the unreacted aldehyde groups were treated with 0.1 M glycine for 30 min.

The piezoelectric crystal biosensor 8 thus formed was calibrated for the determination of C. albicans as follows. The sensor 8 was washed with an 0.5 M NaCl solution and immersed in C. albicans sample solution. After allowing to stand for 30 minutes, the sensor was washed with an 0.5 M NaCl solution again and the resonant frequency $F_2$ was measured. This operation was repeated for different microbial concentrations of C. albicans, each microbial concentration being measured with a hemocytometer. From the resultant measurements of resonant frequency $F_2$, a plot (or calibration curve) (Figure 4) showing the relationship between the resonant frequency and the C. albicans concentration is obtained. The abscissa refers to the concentration of C. albicans while the ordinate refers to the frequency change ($\Delta F = F_1 - F_2$). Each o represents a measured value of $F_2$, and the dotted line represents a resonant frequency change caused by the antibody alone. Thus, it will be seen that the resonant frequency change, $\Delta F$, increases depending on the microbial concentration.

The calibrated biosensor was then operated in a similar fashion, by washing in an NaCl solution, immersing in a C. albicans solution of unknown concentration, washing again and measuring the resultant resonant frequency. The presence of C. albicans was detected by the change of frequency and the concentration determined from the calibration curve or plot previously obtained.

The same biosensor was operated with different microbial concentrations of solutions of Saccaromyces (Saccaromyces cerevisiae (hereinafter referred to as S. cerevisiae)), which is also a yeast. The resonant frequency was measured in the same manner as the one described above and $\Delta F$ plotted against microbial concentrations as shown in Figure 5. The resultant plot showed that no substantial frequency change occurred depending on the S. cerevisiae concentration. Further examination with Escherichia coli and Bacillus were carried out, from which it was found that no substantial change in frequency occurred in these cases as in the case of S. cerevisiae. Thus, the described piezoelectric crystal biosensor has an excellent selectivity for C. albicans.

As a result of an examination of a mixture of C. albicans with S. cerevisiae, it was found that the change in the frequency depends on the concentration of C. albicans alone.

II. Piezoelectric crystal biosensor for detection and concentration determination of biochemicals.

Detection and concentration determination of biochemicals may be effected with high accuracy by incorporating a piezoelectric crystal biosensor, wherein an antibody or an antigen is immobilised on the surface of each electrode, into a flow type cell. In such a piezoelectric crystal biosensor system according to the present invention, the piezoelectric crystal biosensor is incorporated in a flow type cell so that the sensor is in liquid at all times. To avoid unstable oscillation by mechanical pressure, the whole of the piezoelectric crystal is immersed in liquid. Further, because the resonant frequency of the biosensor crystal varies according to the temperature, conductivity and flow rate of the liquid, the measurements of the frequency before and after an antigen-antibody reaction are carried out by replacing solutions with distilled water and feeding distilled water stored in a thermostatically controlled bath to the cell at a constant rate.

The sensitivity of the piezoelectric crystal biosensor may be enhanced if after samples have been exposed to the treated surfaces of the piezoelectric crystal, latexes or other fine particles, on which an antibody or an antigen is immobilised, are passed over the surfaces and are bound by antigen-antibody reaction to the surface of the piezoelectric crystal. Thus, the weight change of the surface of the piezoelectric crystal is amplified with latexes or other fine particles.

EXAMPLE 2

A piezoelectric crystal biosensor was prepared in the following manner. The surface of each electrode 62 (Figure 6) of a piezoelectric crystal 61 was plated with palladium and anodised in 0.5 M NaOH for one hour. Then it was treated in 10% gamma-amino propyl triethoxy silane in acetone for two hours and then in 5% glutaraldehyde for three hours. Subsequently, it was immersed in 1mg/ml solution of protein A to immobilise receptor on the electrode surfaces. It was further immersed in 0.1 M glycine for 30 minutes to treat unreacted aldehyde groups.

The piezoelectric crystal 61 has on each side an electrode 62 on which the receptor is thus immobilised. Each electrode 62 is connected to a lead wire 63 and the biosensor is placed in a cell 64 from which the wires 63 project. The cell 64 also has pipes 65 connected thereto to enable the circulation of liquid therethrough. The wires 63 are connected to an oscillator circuit 66 (Figure 7) which in turn is connected to a frequency counter 67 to which a computer 68 is connected to acquire the data obtained from the frequency counter 67. One of the pipes 65 is connected to a peristaltic pump 69 to extract liquid from the cell 64, while the other or inlet pipe 65 is connected to the outlet of a multi-inlet valve 70. The valve 70 has, in this instance, four inlets any one of which may be selectively connected to the inlet pip 65. One inlet of the valve 70 is connected to a bath 72 for distilled water stored in a thermostatically controlled bath 71. Other inlets, of which only one inlet 73 is shown, are for introducing 0.5 M NaCl, glycine-hydrochloride buffer (pH 2.8) and sample solutions.

The determination of human IgG with the piezoelectric crystal biosensor system was carried out in the following manner. The valve 70 was adjusted and the pump 69 operated, so that a glycine-hydrochloride buffer (pH 2.8) was circulated through the cell to remove adsorbates. Then the valve 70 was changed and the contents of the cell were replaced by distilled water. The oscillation frequency $F_1$ was measured while feeding distilled water at a constant rate. The resonant frequency was monitored continuously until the frequency became constant. Then the valve 70 was again changed and human IgG solution of known concentration was circulated through the cell and allowed to react for 30 minutes at 30° C. Then the valve 70 was changed and 0.5 M NaCl was circulated through the cell to remove non-specific adsorbates. Then the valve 70 was changed and the contents of the cell were replaced by distilled water again and the oscillation frequency $F_2$ was measured while feeding distilled water at the same constant rate. Thereafter, the valve 70 was changed and glycine-hydrochloride buffer (pH 2.8) was circulated through the cell to remove the specific adsorbate for rinse.

This procedure was repeated for other known concentrations of human IgG solutions and the resultant values of resonant frequency $F_2$ were related to oscillation frequency $F_1$ to give the frequency change $\Delta F$. From these a calibration curve was plotted (Figure 8) showing the relationship between the human IgG concentration and the resonant frequency change with the piezoelectric crystal biosensor system on which protein A is used for receptor. The ordinate refers to the resonant frequency change ($\Delta F = F_1 F_2$) while the abscissa refers to human IgG concentration. Thus it is found that the frequency varies depending on human IgG concentration.

It is further found that the frequency returns to the original value after circulating glycine hydrochloride buffer (pH 2.8) through the cell, whereas the frequency further varies by additional reaction when the sensor is used over ten times.

As a result of operation using human albumin instead of human IgG, no response is obtained, which shows that this system responds specifically to human IgG.

When the calibration curve has been established, the biosensor system can be used to detect human IgG in unknown solutions and to determine its concentration.

EXAMPLE 3

A similar result is obtained when non-human IgG antibody is employed as receptor instead of protein A. In this case, a piezoelectric crystal biosensor was prepared in the same manner as the one described in Example 2 except that non-human IgG antibody 91 (Figure 9) was immobilised on electrodes 92 of the piezoelectric crystal.

Glycine-hydrochloride buffer (pH 2.8) was introduced into the cell to remove adsorbates. Then the contents of the cell were replaced by distilled water and the resonant frequency $F_1$ was measured at constant flow rate of distilled water. Subsequently, human IgG solution was introduced into the cell and allowed to react for 30 minutes at 30° C therein. 0.5 M NaCl was passed through the cell to remove non-specific adsorbates. Then the resonant frequency $F_2$ was measured again.

In order to enhance sensitivity, latexes 94 on which nonhuman IgG antibody 91 was immobilised were introduced into the cell and allowed to react for 30 minutes. After passing 0.5 M NaCl again, the resonant frequency $F_3$ was measured. Table 1 shows the response upon IgG ($F_1$-$F_2$) and that enhanced by the latexes ($F_1$-$F_3$), showing that these latexes can enhance sensitivity.

After sufficient solutions of known concentration have undergone this procedure to enable a calibration curve to be produced, unknown solutions may be tested to detect human IgG and determine concentration.

Table 1

| Human IgG conc. (mg/ml) | $F_1$-$F_2$ (Hz) | $F_1$-$F_3$ (Hz) |
|---|---|---|
| $1 \times 10^{-4}$ | 26 | 51 |

EXAMPLE 4

A piezoelectric crystal biosensor was prepared in the same manner as the one described in Example 2. Protein A 101 (Figure 10) was immobilised on the surface of an electrode 102 of a piezoelectric crystal.

Glycine-hydrochloride buffer (pH 2.8) was introduced into the cell to remove adsorbates. Then the contents of the cell were replaced by distilled water and the resonant frequency $F_1$ was measured at a constant flow rate of distilled water. Subsequently, a solution of human IgG 103 of known concentration was introduced into the cell and allowed to react for 30 minutes therein. 0.5 M NaCl was passed through the cell to remove non-specific adsorbates. After replacing the contents of the cell by distilled water, the resonant frequency $F_2$ was measured again. Further latexes 104, on which non-human IgG antibody 103 (mouse $IgG_1$) was immobilised, were introduced into the cell and allowed to react for 30 minutes. After passing phosphate buffer (pH 6 thereby to remove latexes directly bound to protein A, the contents of the cell were replaced by distilled water and the resonant frequency $F_3$ was measured again.

This was repeated for different concentrations of human IgG and the results used to produce a calibration curve.

Comparing the obtained response upon human IgG, i.e. $\Delta F_{1-2}$ ($F_1$-$F_2$) with the one enhanced by the use of latexes, i.e. $\Delta F_{1-3}$ ($F_1$-$F_3$), it was found that $\Delta F_{1-3}$exhibited an enhanced value over the corresponding $\Delta F_{1-2}$, showing that the latexes on which non-human IgG is immobilised can enhance sensitivity.

III. Application to analysis of biochemicals.

The constituents of particular biochemicals are analysed by immobilising receptor materials exhibiting different adsorptivities upon various biochemicals on the surface of a piezoelectric crystal, successively passing eluents differing in properties thereover and determining the change in resonant frequency before and after the elution. An adsorbent receptor, such as selected from among enzymes, sugars, lipids, co-enzymes, amino acids and proteins such as lectins, antibodies or proetin A, is immobilised on the surface of the piezoelectric crystal of a biosensor. A sample to be analysed is adsorbed by the receptor. Then the constituents of the sample are eluted with eluents slightly differing in pH value or ionic strength from each other or containing organic solvents. Thus the presence and amount of each constituent can be determined from the resonant frequency of the piezoelectric crystal biosensor before and after elution.

The constituents of biochemicals can be analysed by the use of a piezoelectric crystal biosenser by taking advantage of the different abilities of adsorbent materials immobilised on the surface of the piezoelectric crystal to adsorb various objects. By proper selection of receptor materials immobilised on the surface and of the eluents used, this sensor has wide application.

EXAMPLE 5

The piezoelectric crystal biosensor was prepared in the same manner as the one described in Example 2, with protein A 111 (Figure 11) immobilised on the surface of an electrode 112 of a piezoelectric crystal.

Glycine-hydrochloride buffer (pH 2.8) was introduced into the cell to remove adsorbates. Then the contents of the cell were replaced by distilled water and the resonant frequency was measured in a constant flow rate of distilled water. Subsequently 0.1 mg/ml mouse gamma-globulin solution in phosphate buffer (pH 8) and containing mouse $IgG_1$ 113, mouse $IgG_{2a}$ 114, and mouse $IgG_{2b}$ 115, was introduced into the cell and allowed to react for 30 minutes while maintaining the cell at 30°C (Figure 11(a)).

Then the contents of the cell were replaced by distilled water (Figure 11(b)) and the resonant frequency $F_0$ was measured. Subsequently it was successively eluted with phosphate/citrate buffer solutions of pH 7.0, 6.5, 6.0, 5.5, 5.0, 4.5, 4.0, 3.5, and 3.0 and the resonant frequency was measured following each elution. As mouse $IgG_1$ is eluted first, followed by mouse $IgG_{2a}$and mouse $IgG_{2b}$, the progress of the operation is illustrated in Figures 11(c), 11(d) and 11(e).

Figure 12(a) shows the resonant frequencies thus measured. Figure 12(b) is a graph which shows the difference in the resonant frequencies before and after each elution measured from the result as shown in Figure 12(a), and shows that there are three obvious peaks. These peaks correspond to $IgG_1$, $IgG_{2a}$ and $IgG_{2b}$, respectively. This result well agrees with the one obtained by affinity chromatography with the use of protein A immobilised gel column, PL. Ey et al, Immunochemistry, 15 (1978) 429.

EXAMPLE 6

The constituents of human IgG were analysed in the same system and in the same manner as those described in Example 2. Namely, a piezoelectric crystal biosensor on which protein A was immobilised was used as in Example 2. As in Example 2, glycine hydrochloride buffer solution (pH 2.8) was introduced into the cell to thereby remove adsorbates. Then the content of the cell was replaced with distilled water and the resonant frequency was measured at constant flow rate of distilled water. Subsequently 0.1 mg/ml solution of human gamma-globulin in phosphate buffer solution (pH 7) was introducted into the cell and allowed to react for 30 minutes while maintaining the cell to 30°C. Then the contents of the cell were replaced with distilled water and the resonant frequency was measured. Subsequently it was successively eluted with phosphate/citrate buffer solutions of pH 6.5, 6.0, 5.5, 5.0, 4.5, 4.0, 3.5, 3.0, and 2.5 and the resonant frequency was measured after each elution. Figure 13 is a graph of the resonant frequencies difference before and afterthe elution determined from a change in the resonant frequency. This result shows a similar tendency to that reported by R.C. Duhamel, P.H. Schur and K. Brendel (cf. J. Immunol. Methods, 31, 211 (1979)), i.e. showing the elution of $IgG_2$ and $IgG_4$ around pH 4.7 and that of $IgG_1$ and $IgG_4$ around pH 4.3.

In Examples 5 and 6, protein A was employed alone as a material which can adsorb biochemicals. However, two or more materials which can adsorb the biochemicals to be analysed may be immobilised on the biosensor crystal electrode. In such a case, each biochemical may be analysed by selecting appropriate eluents to take advantage of the difference in the ability of the materials immobilised on the surface of the piezoelectric crystal electrode to adsorb the biochemicals to be analysed.

IV. Analysis of biochemicals, microorganisms or cells by using a plurality of piezoelectric crystal biosensors.

A plurality of biochemicals can be analysed by an analytical system equipped with a plurality of piezoelectric crystal biosensors, each having a different material immobilised or chemically treated on its surface. The analysis can be accelerated by using as a referential piezoelectric crystal sensor, one in which no material is immobilised on the crystal surface, and measuring the difference of resonant frequency of the referential piezoelectric crystal sensor and the piezoelectric crystal biosensors having immobilised receptors. This removes deviations due to short period aging and changes in the conductivity and temperature of the sample solution.

EXAMPLE 7

The multiple piezoelectric crystal biosensor analytical system illustrated in Figure 16 is an ABO blood type sensor. Four biosensors are used, in each of which a piezoelectric crystal 161 has surface electrodes 162 connected by leads 163 to an oscillator circuit 166 which is connected to a frequency counter 167 connected to a computer 168. The four piezoelectric crystals 161 are mounted in a cell 164 having inlet and outlet pipes 165, the latter being connected to a peristaltic pump 169 to enable circulation of liquid through the cell. The inlet pipe 165 is connected to the outlet of a solenoid valve 1610. The inlets of the solenoid valve are connected to another solenoid valve 1611 and to a sample inlet 1612. The inlets of the solenoid valve 1611 are connected to a reservoir 1613 of 0.05 M phosphate buffer solution (pH 7.5) and a reservoir 1614 of 0.1 M alpha-L-fucose and 0.1 M N-acetyl-D-glucosamine. The frequency counter 167, peristaltic pump 169 and solenoid valves 1610 and 1611 are connected to the computer 168 by which the system is controlled.

Each piezoelectric crystal 161 was prepared in the following manner. The surface of each electrode 162 of the piezoelectric crystal 161 was plated with palladium and treated anodically to oxidise in 0.5 M NaOH for one hour. Then it was treated in acetone solution containing 5% trisyl chloride and 1.2% pyridine for 30 minutes and washed with 5mM HCl. One crystal was left untreated. Each of the other three wastreated to immobilise a different lectin on its surface electrodes. The lectins were Phaseolus Limensis Agglutinin, Bandeiraea Simplicifolia Lectin I and Ulex Europaeus Agglutinin I. Each lectin was immobilised by immersing the piezoelectric crystal in 1 mg/ml lectin solution for one hour. It was further reacted in 0.1 M glycine solution for 30 minutes thereby to treat unreacted aldehyde groups.

ABO blood type determination by this device was carried out in the following manner.

The three piezoelectric crystals, each having a different lectin immobilised thereon, and the referential untreated piezoelectric crystal were previously resonated in the cell through which buffer solution from reservoir 1613 was passed. Then the solenoid valve 1610 was switched thereby to introduce blood cell sample via the sample inlet 1612. The sample was arbitrarily diluted by adjusting the solenoid valve 1610. When the sample was passed through the cell, the peristaltic pump 169 was controlled so as to give a low flow rate to secure sufficient progress of the reaction. Then phosphate buffer (pH 7.5) was passed therethrough to remove non-specific adsorbates. The resonant frequency of each piezoelectric crystal was measured. The differences between the resonant frequencies of the piezoelectric crystal on which one of the three lectins was immobilised and that of the referential piezoelectric crystal were measured. The blood type was then determined depending on a change in the frequency before and after the reaction with the sample.

As a result, it was found that the piezoelectric crystal on which Phaseolus Limensis Agglutinin was immobilised would respond only to erythrocytes having type A factor. On the other hand, the one on which Bandeiraea Simplicifolia Lectin I was immobilised would respond to both type A and type B factors, but show a higher response to the latter. Thus the blood types A, B and AB can be distinguished from each other by comparing the responses with those of the piezoelectric crystal on which Phaseolus Limensis Agglutinin was immobilised. In contrast, the piezoelectric crystal on which Ulex Europaeus Agglutinin I was immobilised would respond only to type O erythrocytes.

Then the solenoid valve 11 was switched to introduce 0.1 M alpha-L-fucose and 0.1 M N-acetyl-D-glucosamine (pH 7.5) into the cell. Thus erythrocytes bound to the surface of the lectins were removed. Subsequently, a sufficient amount of phosphate buffer (pH 7.5) was passed through and the determination was performed again.

As described above, the analytical device of the present invention can accelerate continuous judgement of ABO blood types.

As shown in Figure 14, the multiple piezoelectric crystal biosensor cell may comprise piezoelectric crystals 141 of AT-cut 9 MHz. An electrode 142 is provided on each side of each piezoelectric crystal 141 and a lead 143 is connected thereto. A cell 144 surrounds the piezoelectric crystals 141 and has pipes 145 to permit circulation of liquid therethrough. The leads 143 project from the cell 144.

A smaller cell can be used in a device which GT-cut piezoelectric crystals are employed, which brings out an additional advantage that only a small amount of a sample is required. This is illustrated in Figure 15 in which four piezoelectric crystals 151 of GT-cut 4MHz have electrodes 152 connected by leads 153 to the exterior of a common cell 154 surrounding the crystals. Pipes 155 permit circulation of liquid through the cell 154.

**Claims**

1. An analytical device for biochemicals, microbes and cells, comprising a piezoelectric crystal sensor (1,2; 61,62; 141,142, 151,152; 161,162) and means (6,7; 66,67; 166,167) for measuring the resonant frequency of the sensor, the sensor exhibiting a resonant frequency change caused by weight change on the surface of the piezoelectric crystal due to the biochemical, microbe or cell to be analysed.

2. A device according to claim 1, in which the sensor has receptor material immobilised on or chemically treated onto the surface of the piezoelectric crystal.

3. An analytical device for biochemicals, microbes and cells, comprising a plurality of piezoelectric crystal sensors (141,142; 151,152; 161,162) and means (166,167) for measuring the resonant frequencies of the sensors at least one of the sensors exhibiting a resonant frequency change caused by weight change on the surface of the piezoelectric crystal due to the biochemical, microbe or cell to be analysed.

4. A device according to claim 3, in which at least two of the piezoelectric crystals have different receptor materials immobilised on or chemically treated onto their surfaces.

5. A device according to claim 3 or 4, in which at least one piezoelectric crystal is free from any receptor material immobilised on or chemically treated onto the surface thereof.

6. A device according to claim 2 or 4, in which biochemical receptor material is selected from sugars, lipids, co-enzymes, amino acids and proteins including enzymes, lectins, antibodies and protein A.

7. A device according to any preceding claim in which the measuring means comprises an oscillator circuit (6; 66; 166) and a frequency counter (7; 67; 167).

8. A method for the analysis of biochemicals, microbes and cells, comprising (a) applying adsorbent biochemicals or organic compunds to a piezoelectric crystal biosensor surface; (b) allowing the biochemicals or organic compund to adsorb biochemicals, microbes or cells to be analysed; (c) measuring the resonant frequency change caused by a weight change on the piezoelectric crystal surface; and (d) detecting the biochemicals, microbes or cells, and determining concentration of the biochemicals, microbes or cells from a calibration curve obtained previously.

9. A method according to claim 8, in which the piezoelectric crystal is incorporated in a flow cell.

10. A method according to claim 8 or 9, in which the whole piezoelectric crystal is immersed in liquid.

11. A method according to claim 8, 9 or 10 in which the measurement is carried out whilst the crystal is subject to a flow of substantially constant temperature distilled water.

12. A method according to claim 8, 9, 10 or 11 in which fine particles, on which a biochemical or organic compound is immobilised, are applied to attach to the adsorbed biochemicals, microbes or cells on the piezoelectric crystal biosensor surface.

13. A method according to claim 12, in which the fine particles are of latex.

14. A method according to any of claims 8 to 13, in which the biochemical or organic compound is an antibody or antigen.

15. A method for the analysis of biochemicals, microbes and cells using a piezoelectric crystal biosensor, comprising immobilising at least one material which can adsorb biochemicals, microbes or cells on the surface of the piezoelectric crystal, allowing immobilised material to adsorb the biochemicals, microbes or cells to be analysed, and analysing the constituents of the biochemicals, microbes or cells on the basis of the ability of the immobilised material to adsorb the biochemicals, microbes or cells.

16. A method for the analysis of biochemicals using a piezoelectric crystal biosensor, comprising immobilising at least one material which can adsorb biochemicals on the surface of the piezoelectric crystal, allowing the immobilised material to adsorb the biochemical to be analysed, flowing an eluent over the surface and analysing the constituents of the biochemical from a change in the resonant frequency of the crystal before and after the elution.

17. A method according to claim 16, in which the immobilised material exhibits different adsorptivities with different biochemicals, in which eluents with differing properties are successively flowed thereover and in which analysis of the constituents of the biochemical is made on the basis of the difference in the adsorptive ability of the immobilised material to adsorb different biochemicals.

18. A method according to claim 17, in which the eluents are selected fromamong those differing in pH or ionic strength from each other, and containing materials which prevent the adsorption or containing organic solvents.

F I G. 1

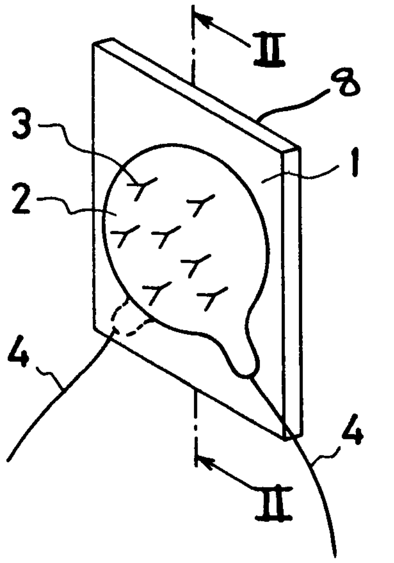


F I G. 2

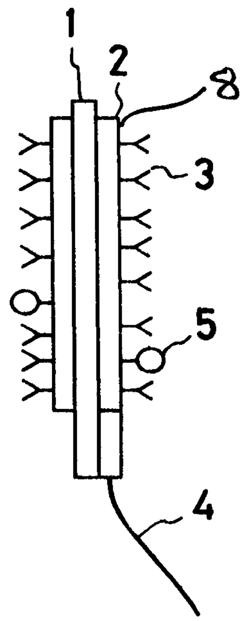


F I G. 3

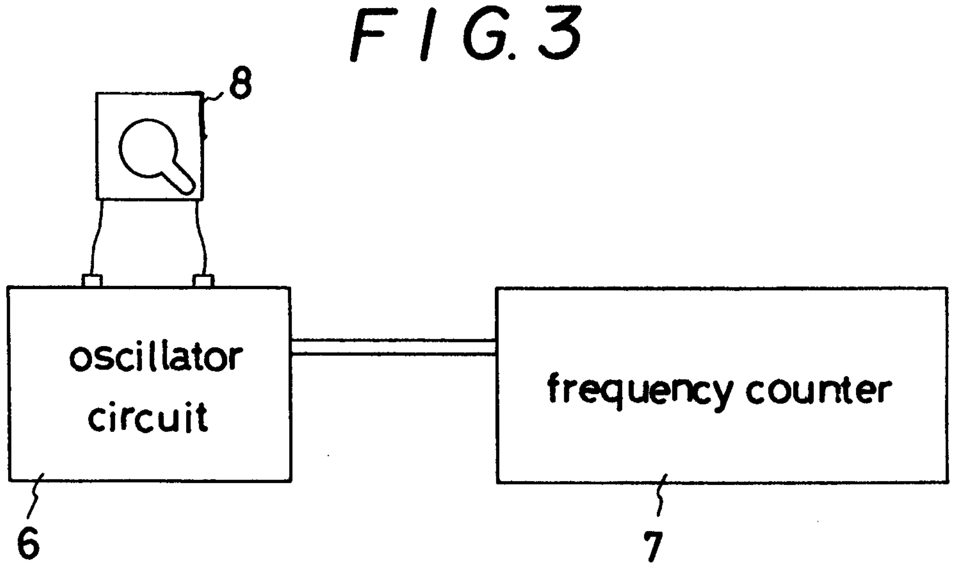

FIG.4

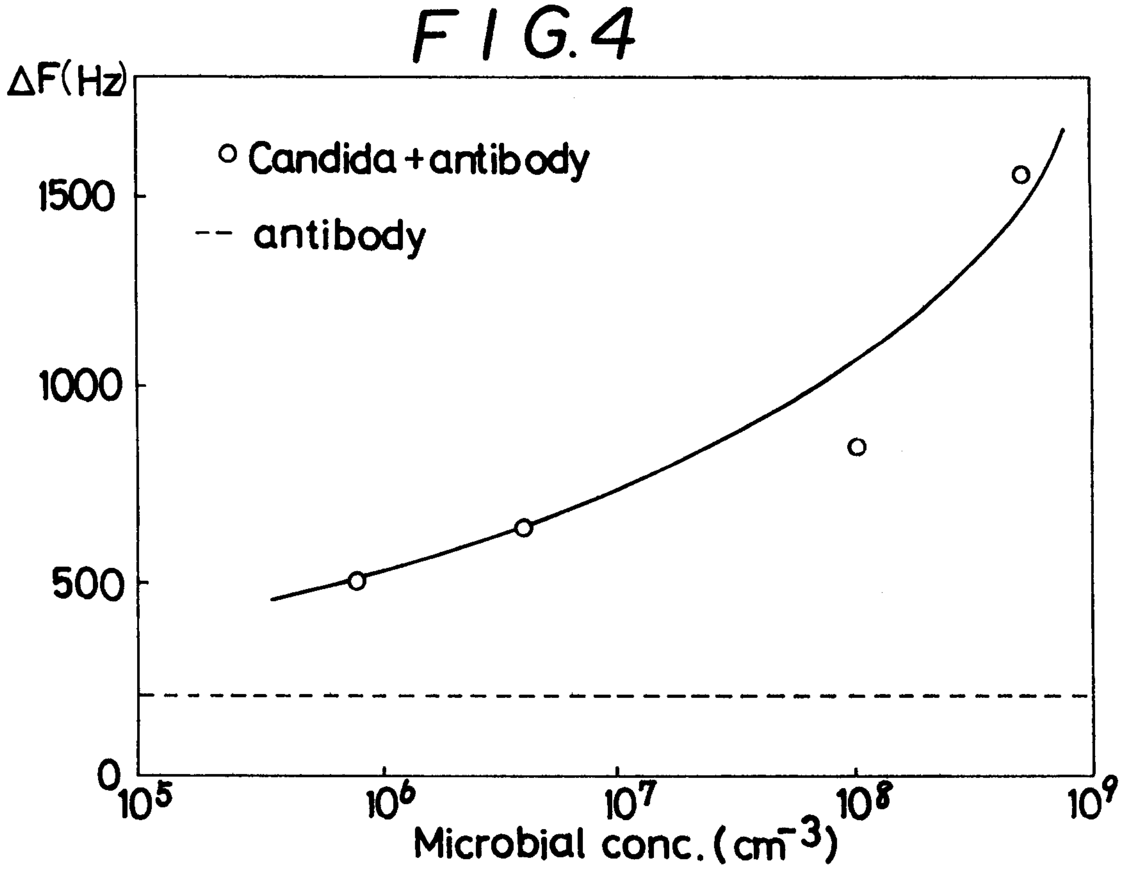
ΔF(Hz)
○ Candida + antibody
-- antibody
1500
1000
500
0
$10^5$
$10^6$
$10^7$
$10^8$
$10^9$
Microbial conc. ($cm^{-3}$)

FIG.5

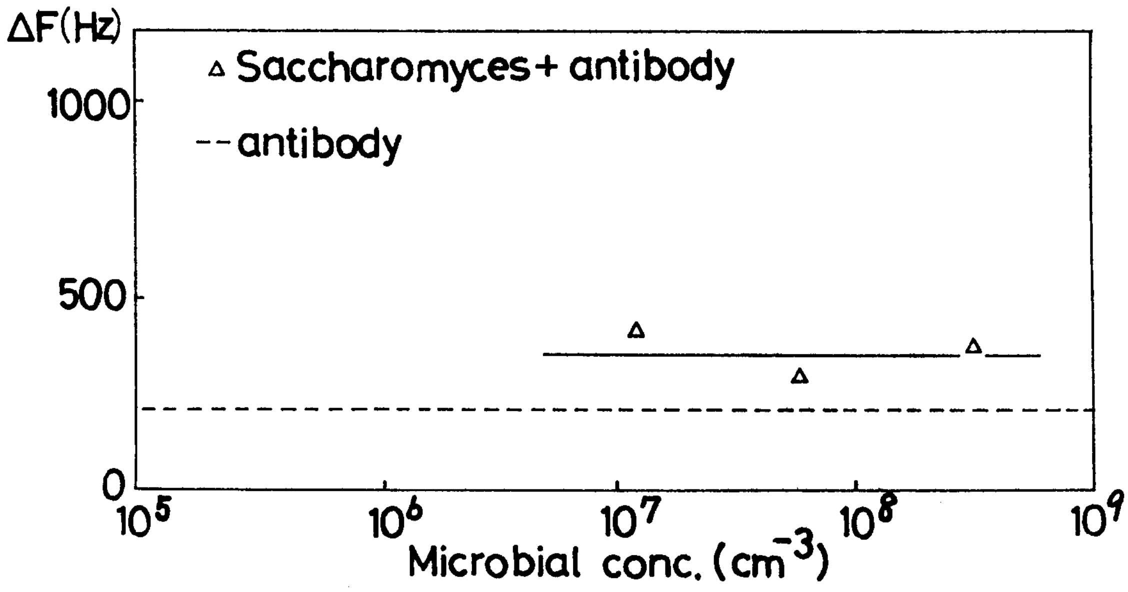
ΔF(Hz)
△ Saccharomyces + antibody
-- antibody
1000
500
0
$10^5$
$10^6$
$10^7$
$10^8$
$10^9$
Microbial conc. ($cm^{-3}$)

FIG. 6

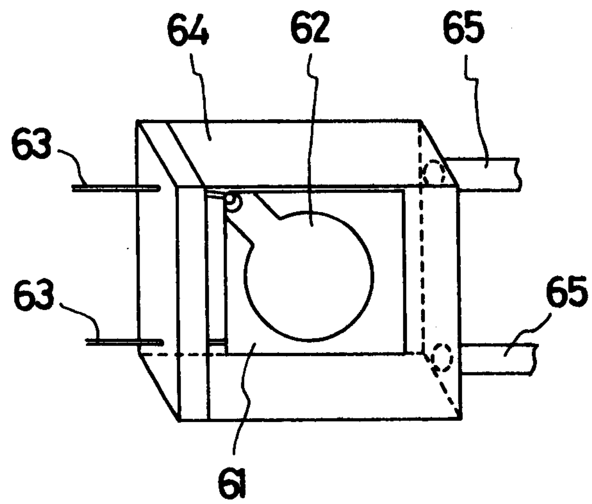
64
62
65
63
63
65
61

FIG. 7

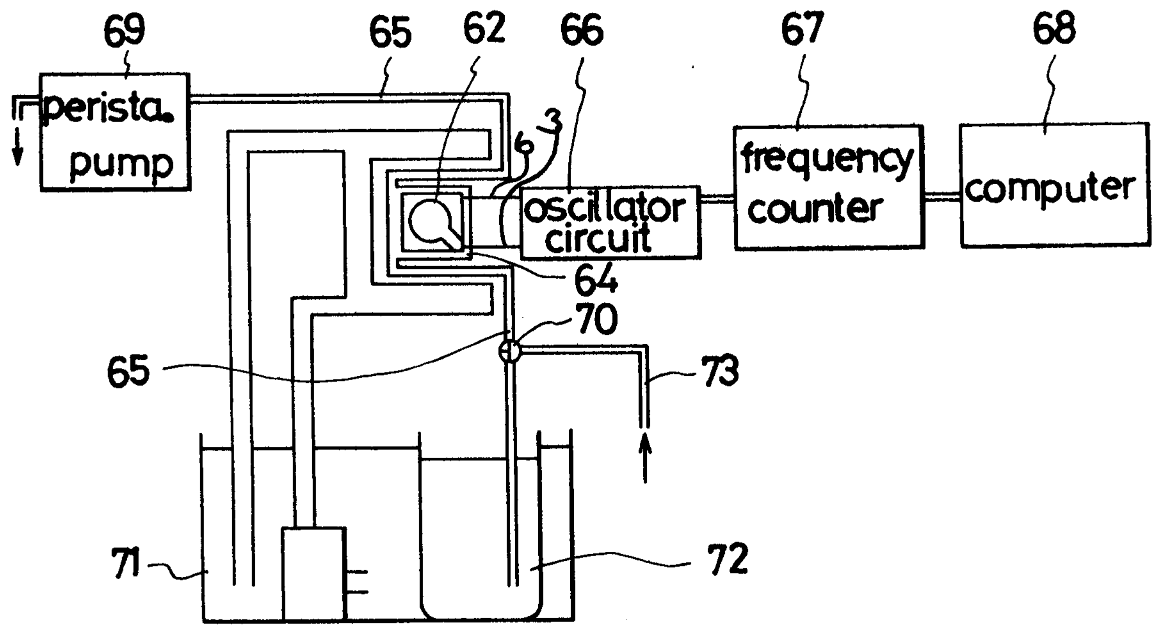
69
65
62
66
67
68
perista. pump
oscillator circuit
frequency counter
computer
63
64
70
65
73
71
72

FIG. 8

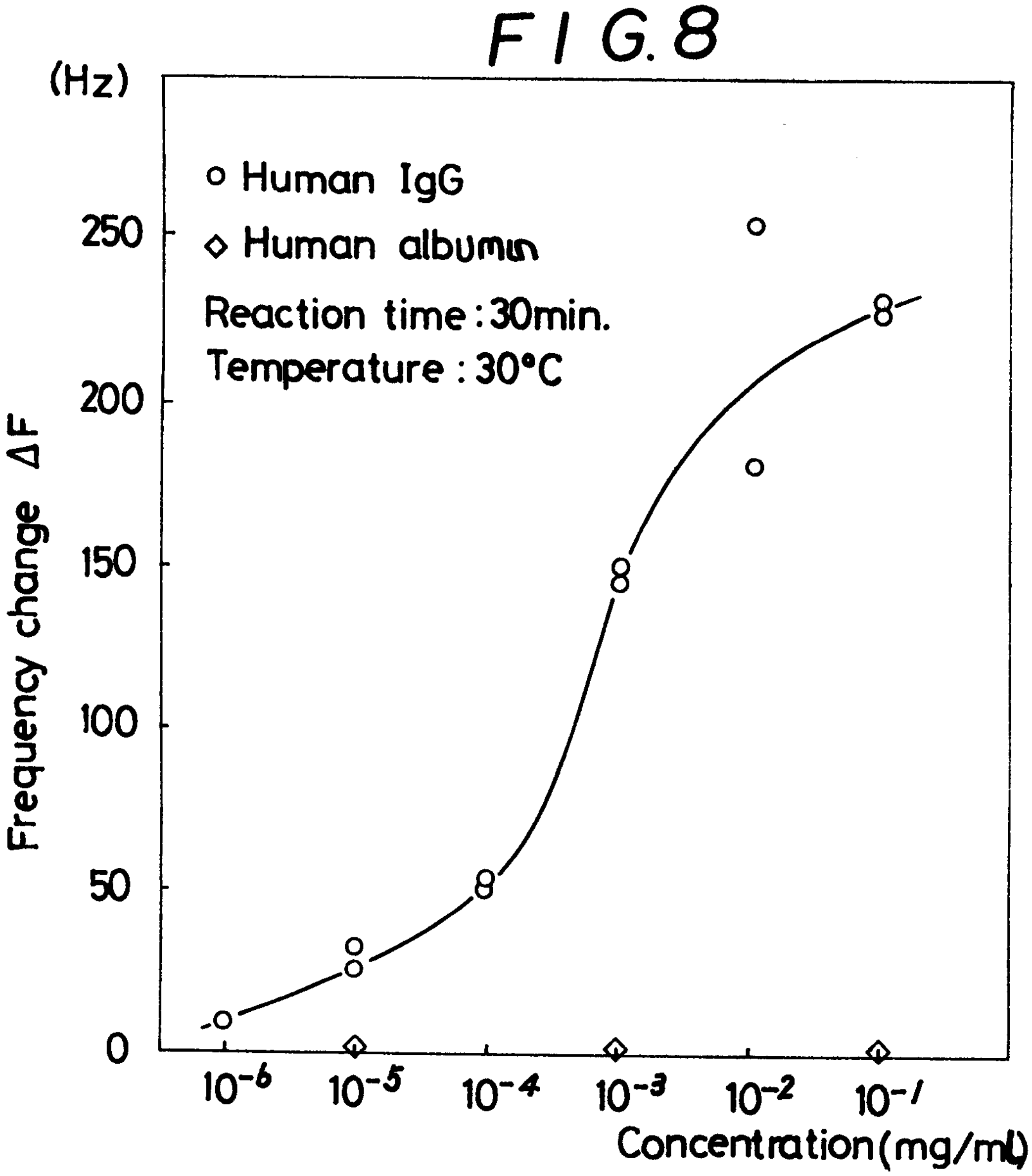
(Hz)
○ Human IgG
◇ Human albumin
Reaction time : 30min.
Temperature : 30°C
250
200
150
100
50
0
Frequency change ΔF
10^-6
10^-5
10^-4
10^-3
10^-2
10^-1
Concentration(mg/ml)

FIG. 9

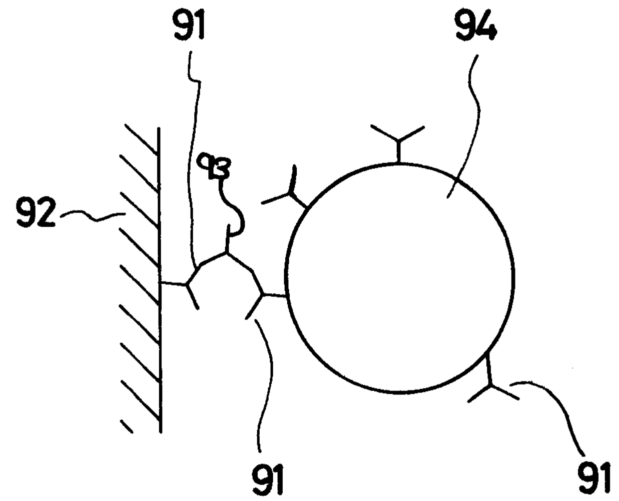


FIG. 10

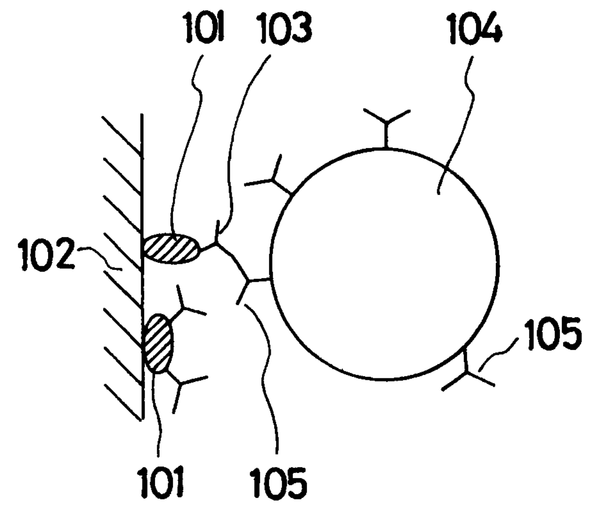

FIG.11

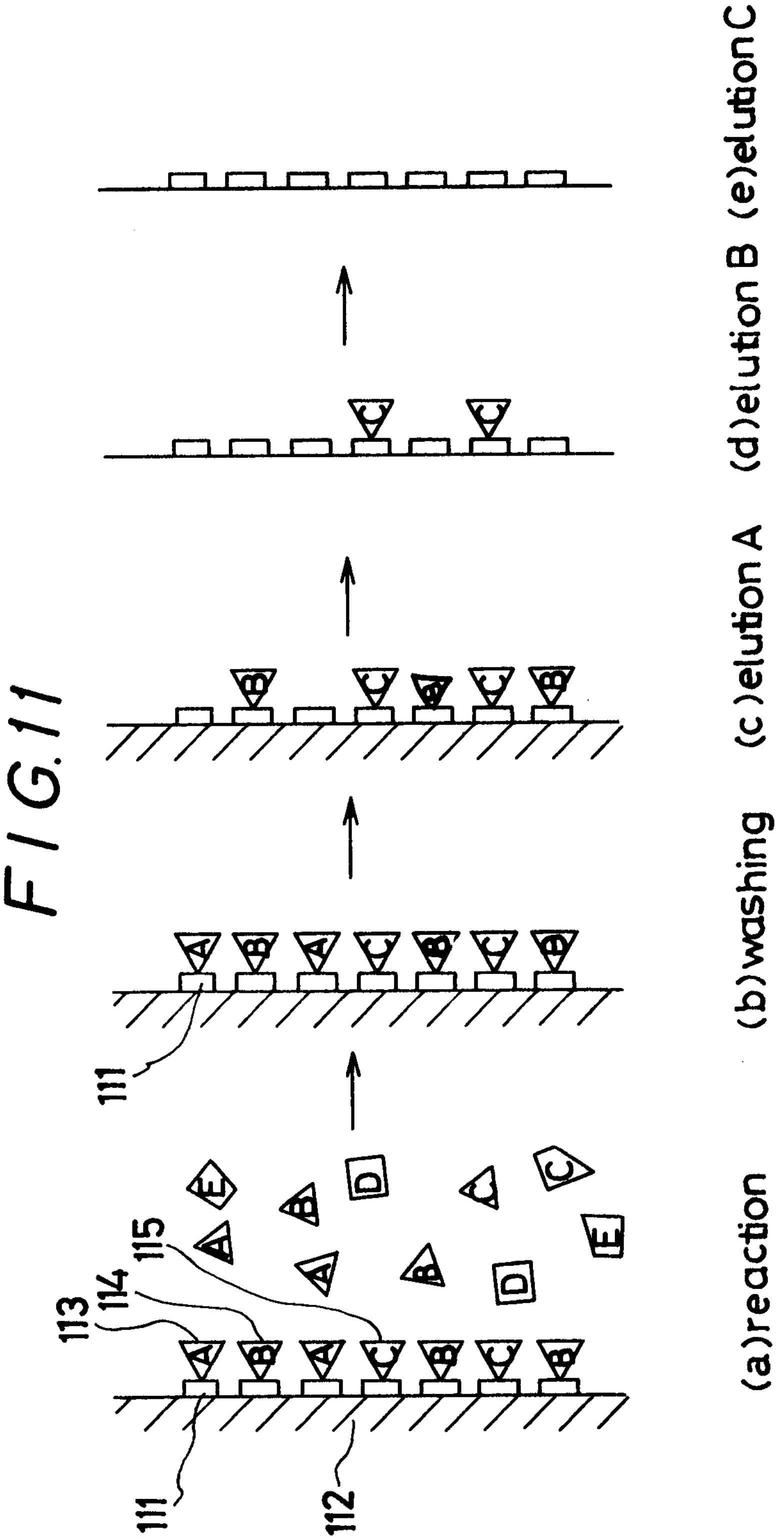
111
112
113
114
115
111
(a)reaction
(b)washing
(c)elution A
(d)elution B
(e)elution C

# FIG.12 (a)

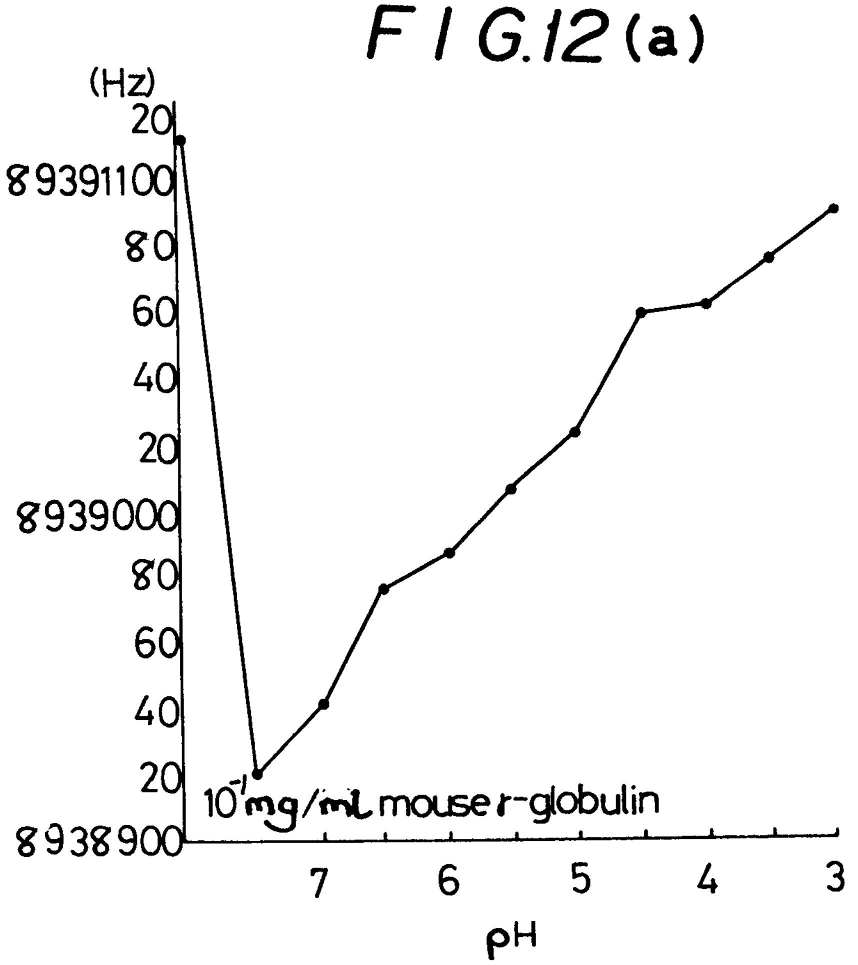


# FIG.12 (b)

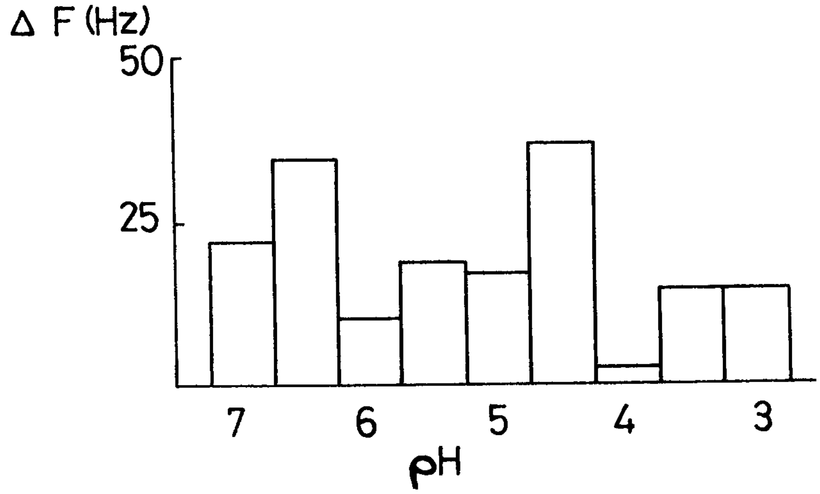

FIG.13

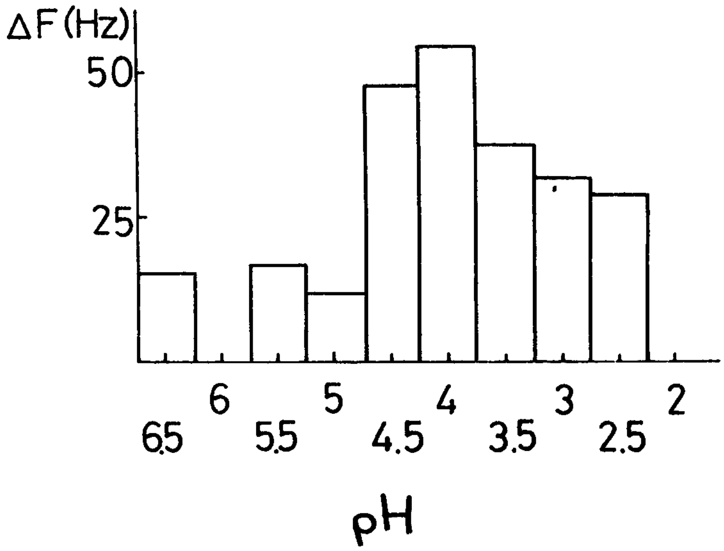


FIG.14

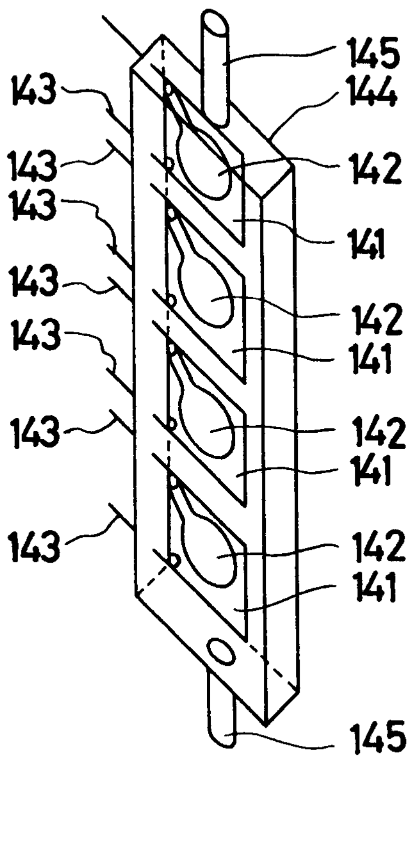


FIG.15

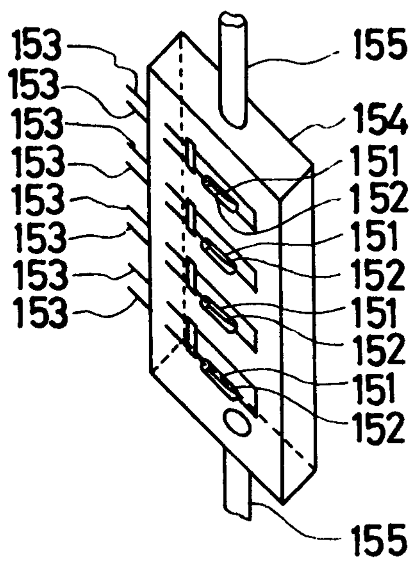

FIG.16

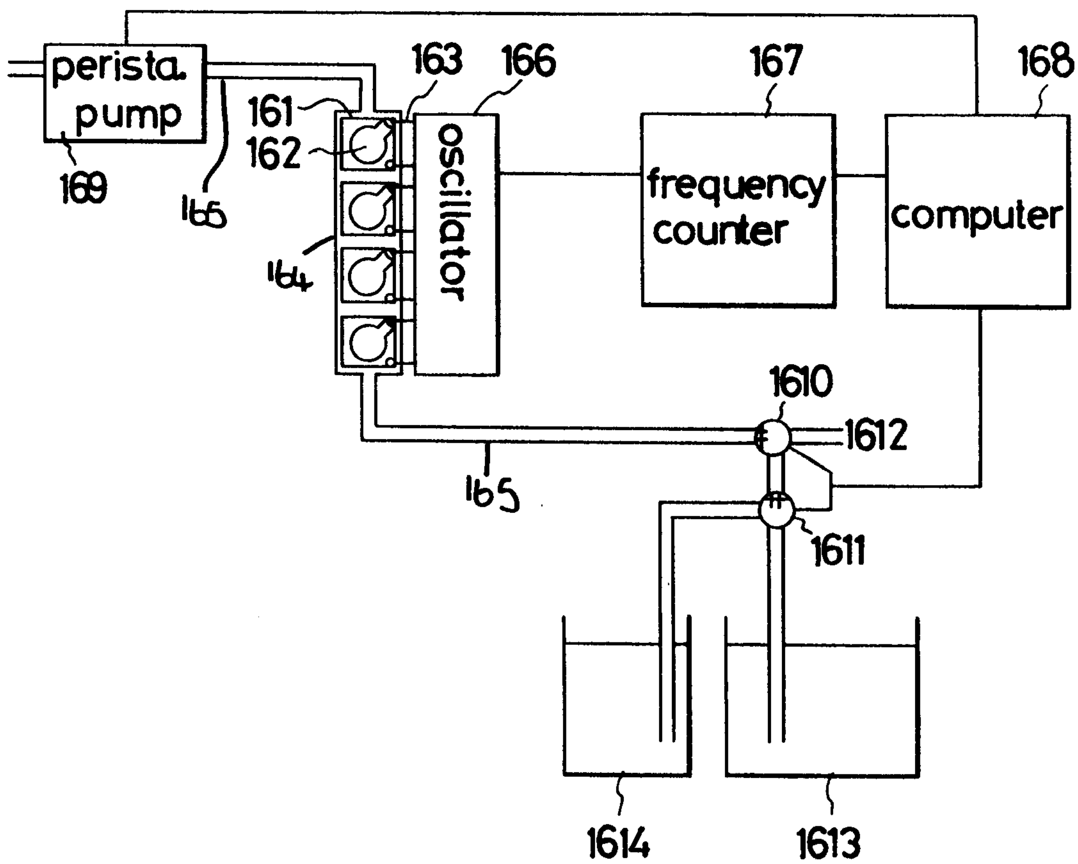
perista. pump
169
165
161
162
163
164
166
oscillator
167
frequency counter
168
computer
1610
1612
165
1611
1614
1613